# EUROPEAN PATENT SPECIFICATION

(11) **EP 1 998 691 B1**
(45) Date of publication and mention of the grant of the patent: **18.08.2010**
(21) Application number: 07754399.9
(22) Date of filing: 28.03.2007
(51) Int. Cl.: A61B 17/17

(54) **LOCKING BONE PLATES WITH CONTROLLED LOCKING SCREW MISALIGNMENT**
FIXIERENDE KNOCHENPLATTEN MIT GESTEUERTER FIXIERSCHRAUBEN-FEHLAUSRICHTUNG
BLOCAGE DE PLAQUE VISSÉE AVEC DÉSALIGNEMENT CONTRÔLÉ DES VIS DE BLOCAGE

(30) Priority: 28.03.2006 US 786390 P
(43) Date of publication of application: 10.12.2008
(73) Proprietor: Synthes GmbH, 4436 Oberdorf (CH)
(72) Inventor: DELL'OCA, Alberto, Fernandez, Montevideo, 11500 (UY)
(74) Representative: Lusuardi, Werther
(86) International application number: PCT/US2007/007874
(87) International publication number: WO 2007/123655

(56) References cited:
- WO-A-00/47120
- US-A1- 2003 083 667
- US-A1- 2006 009 771

## Description

### Field of the Invention

The invention relates generally to implanted bone plate systems for fixing bone fractures. More particularly, the invention relates to locking bone plates with locking screws that are intentionally and controllably misaligned angularly through holes in the bone plate.

### Background of the Invention

Since the early 20th century, bone plates and screws have been used for internal fixation of broken bones. As of the late 1980's, locking bone plates were developed. A locked bone plate uses a locking screw that has threads on an outer surface of its head that mates with corresponding threads in the bone plate hole. Because of the fixed relationship between locking screws and the bone plate, locking screws provide high resistance to shear or torsional forces. Thus, the main feature of such "locking bone plates" is a solid fixation between the plate and inserted screws. The advantages of locked plates - angular stability, less bone vascular damage, better infection resistance - became evident. Since then, use of locked plates has exploded, and they are now produced by different manufacturers.

Different systems have been developed to solidly lock the screw head to the plate hole. In most of these known devices, the locked screw has to be inserted at a predetermined angle. Should the surgeon insert the locking screw at a different angle, either the screw will not lock, or the screw will only lock provisionally, providing little or no angular stability and ultimately giving way under load. (Although there is an angular screw tolerance of some degrees, which can vary from system to system depending on the manufacturer, staying within that tolerance is difficult). An example of such a device is disclosed by Tepic in U.S. Patent No. 5,151,103.

Other disadvantages of known locking systems and insertion procedures include jamming of the screw head in the plate hole (considered by some as cold welding), which has often become a nightmare when the surgeon needs to remove the locked screw. Sometimes the surgeon was forced to cut apart the plate within the patient in order to remove the locked screw. This can result in serious tissue damage and put the internal fixation at considerable risk.

In order to remedy these disadvantages, various changes to known bone plate systems were made, including changes to the design of the plate hole and/or screw head, the precision of the insertion technique of the screw, the amount of insertion torque used, and the type of metals used to form the screws and plates, among others. However, these changes have failed to provide an adequate solution to the disadvantages described above.

Therefore, a need still exists to provide devices and systems having the advantages of locked plates, while preventing excessive locking and allowing reliable and safe removal of the locking screw, if needed.
From US-A 2003/0083667 by RALPH a bone plate is known with plate holes, a drill guide body with a conical bore, a drill guide collet with a longitudinal bore for receiving a drill bit and a drill guide stem, wherein during drilling the drill guide collet is inserted in the conical bore such that the collet shaft can be angled with respect to the central axis of the plate hole.
From US 2006/0009771 by ORBAY a bone plate is known with a set of locking screw holes each having an internal thread. The threaded plate holes are preferably cylindrical threaded holes.

It is an object of the invention to provide a locking bone plate device and system that permits safe and reliable removal of the locking screw, should it be needed, while maintaining the advantages of locking plates, including angular stability, less bone vascular damage, and better infection resistance, among others.

The invention addresses the main reasori why locking screws are often jammed in the plate: the three-dimensional geometrical problem of a too perfect match among the threads in the plate hole and on the locking screw.

Generally, the better the surgical technique, the more perfect the position of the locking screw with respect to the plate hole, the more stable the assembly -- but, on the other hand, the more difficult it may become to remove the screw afterwards, should it be required, because of the likelihood of jamming.

The invention therefore includes an advantageously conical drilling sleeve that guides a drill bit though a bone plate to drill a hole in bone. The drilled hole has an axis which differs slightly (i.e., does not coincide with) the axis of the bone plate hole. The drilling sleeve engages the locking bone plate and guides a drill bit to drill a hole for a locking screw.

The conical drilling sleeve of the invention guides the drill bit so as to drill a bone hole of which the axis does not exactly coincide or angularly align with the axis of the plate hole. However, even though the axes differ slightly, the axis of the drilled bone hole is still at or within the tolerance angle required to maintain good mechanical performance of locked implants.

A locking screw is then inserted into the drilled bone hole, the axis of the locking screw thus being slightly different than the hole axis, yet this difference is still within the tolerance required to maintain proper mechanical performance of the screw-plate coupling. This insertion technique, referred to as "controlled misalignment," provides satisfactory mechanical performance while avoiding the jamming between then plate hole and the locking screw. This permits the surgeon to reliably and safely remove the locked screw, when necessary.

### Brief Description of the Drawings

The objects and advantages of the invention will be apparent upon consideration of the following detailed description, taken in conjunction with the accompanying drawings, in which like reference characters refer to like parts throughout, and in which:

FIG. 1 is a perspective view of a known bone plate system;

FIG. 2 is a perspective view of the known system of FIG. 1 showing a conventional cylindrical sleeve threaded into the threaded bone plate hole with a drill bit having drilled a hole into bone;

FIG. 3 is a cross-sectional view of the known system of FIG. 1 after removal of the drill bit and conventional sleeve showing the drilled bone hole having a central axis that coincides with the central axis of the plate hole;

FIG. 4 is a cross-section of the known system of FIG. 1 showing a bone plate screw perfectly screwed into the threaded bone plate hole;

FIG. 5 is a perspective view of a bone plate system according to the invention;

FIG. 6 is a perspective view of the system of FIG. 5 showing the conical sleeve threaded into the threaded bone plate hole with a drill bit having drilled a hole into bone;

FIG. 7 is a cross-sectional view of the system of FIG. 5 after removal of the drill bit and conical sleeve showing the bone hole drilled in the controlled misalignment position; and

FIG. 8 is a cross-sectional view of the system of FIG. 5 showing a bone plate screw imperfectly screwed into the threaded bone plate hole according to the invention.

### Detailed Description of the Invention

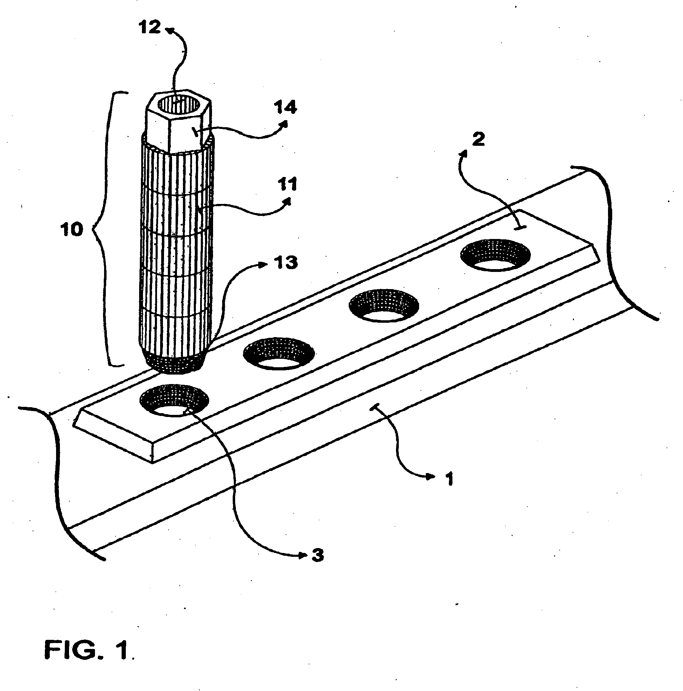

FIGS. 1-4 illustrate a conventional locking bone plate system that includes a conventional locking plate 2, a conventional cylindrical drilling sleeve 10, and a conventional locking screw 4.

Conventional bone plate 2 typically has at least two threaded holes, which in this example are conical threaded holes 3, designed to receive locking screws 4. Locking screws 4 have a threaded head 5, which in this example is conically shaped, and a threaded shaft 6 extending from head 5. The external threads on threaded head 5 mate with and preferably perfectly match the female threads in each of plate holes 3.

Conventional cylindrical sleeve 10 has a threaded head 13 that also mates with and preferably perfectly matches the female threads in each of plate holes 3.

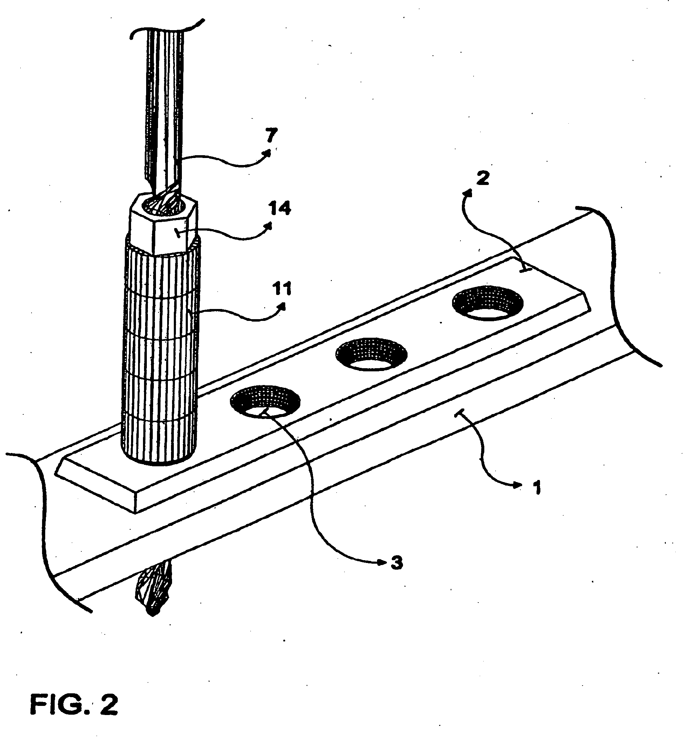

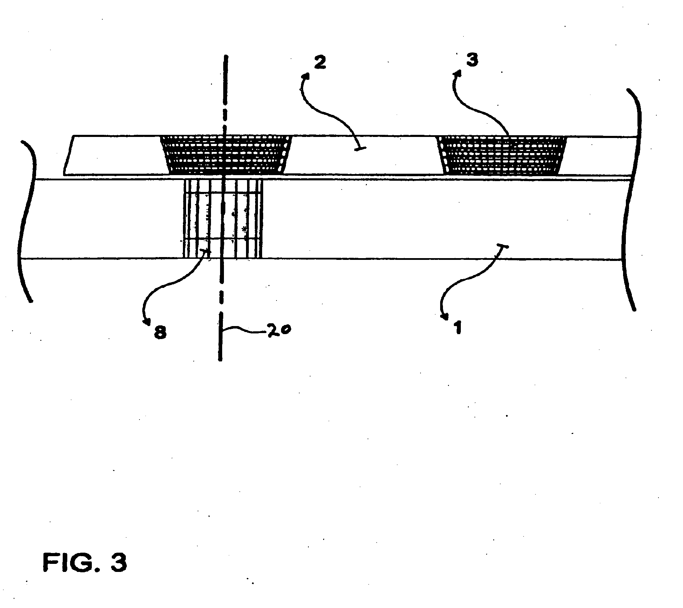

Once the conventional cylindrical sleeve 10 is properly positioned and screwed into one of plate holes 3, as shown in FIG. 2, bore 12 in cylindrical body 11 of conventional sleeve 10 is operative to guide drill bit 7 there through to drill a hole 8 in bone 1. Hole 8 will have the same central axis 20 as plate hole 3, as shown in FIG. 3.

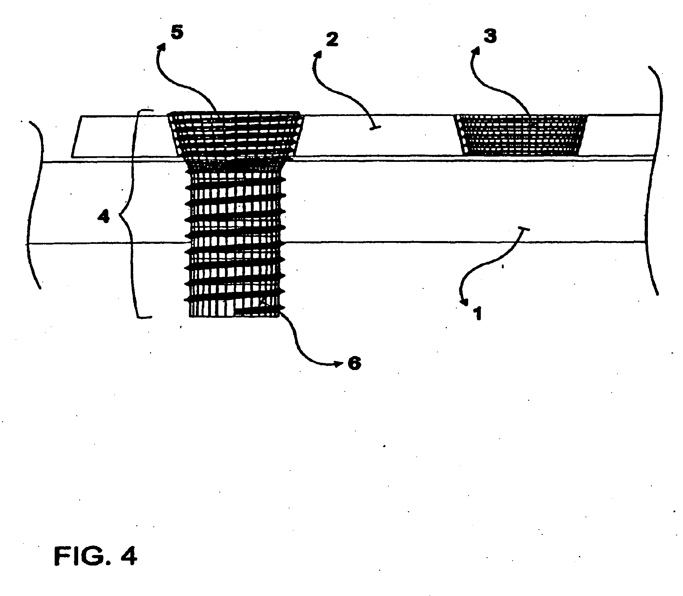

FIG. 4 shows the so-called "perfect position and orientation" of locking screw 4 screwed into plate hole 3 and drilled hole 8, with the central axis of the locking screw coincident with the central axes of the plate hole and drilled hole. In other words, the thread on head 5 of locking screw 4 is offset from and exactly parallel to the thread of plate hole 3, resulting in the "perfect" engagement of the threads in the head of screw 5 with the threads of plate hole 3.

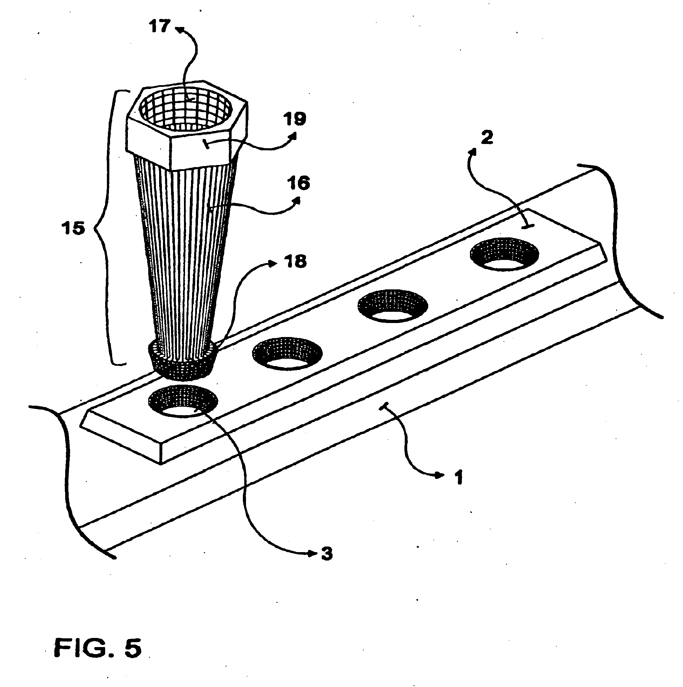

FIGS. 5 to 8 refer to a preferred embodiment of the bone plate system of the invention, which includes a locking bone plate 2 and a conical drilling sleeve 15. Conical drilling sleeve 15 engages locking plate 2 to guide a drill bit 7 there through for drilling a bone hole 9 to receive a locking screw 4.

In particular, locking plate 2, which may be a conventional locking bone plate, preferably has at least two threaded holes, which in this embodiment are conical threaded holes 3, designed to receive locking bone screws 4. Locking bone screws 4 have a threaded head 5, which in this embodiment is conically shaped to engage the female thread of any one of plate holes 3. Locking bone screws 4 also have a threaded shaft 6 extending from head 5 for engaging bone.

As shown in FIG. 5, conical drilling sleeve 15 has a threaded head 18 at its front end and a hexagonal head 19 at its rear end. Hexagonal head 19 is designed to accommodate a wrench, and threaded head 18 mates with and preferably perfectly engages the female thread at each plate hole 3. The cone angle is such that, when conical sleeve 15 is engaged in a plate hole 3, the angle between the inner cone wall in bore 17 and the central axis of plate hole 3 is equal to or less than the tolerance angle (which may differ between different manufacturers of locked bone plates and screws). The tolerance angle is the maximum angle as measured from the central axis of the threaded plate hole at which a bone plate screw can be positioned at the plate hole and the threaded head of the bone plate screw still screwed into and out of the threaded hole.

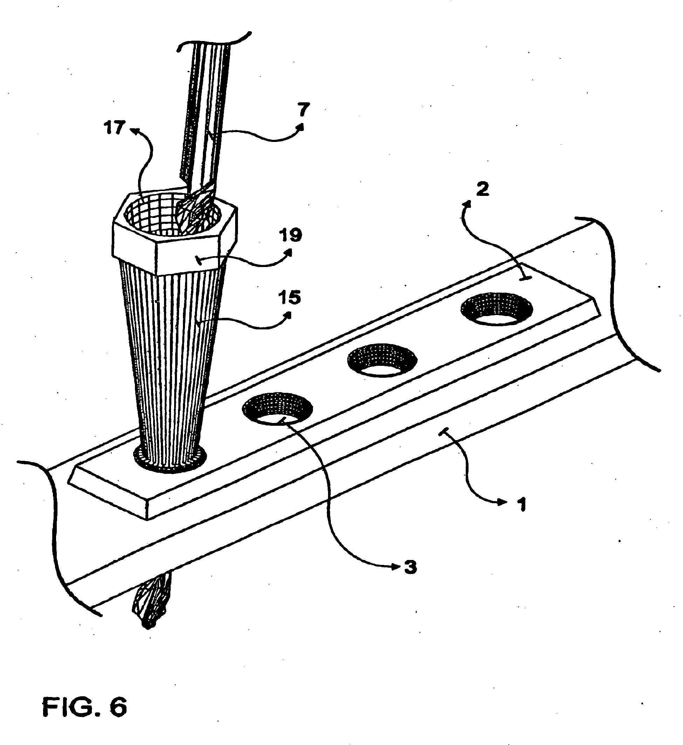

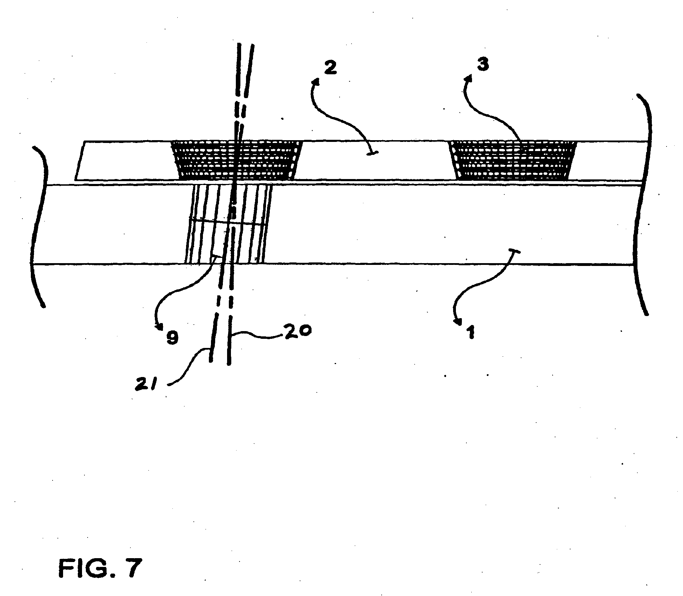

Once the conical sleeve 15 is perfectly positioned in a plate hole 3, drill bit 7 is inserted through conical bore 17 of sleeve 15 such that the drill bit preferably contacts and is guided by the inner wall of conical bore 17, as shown in FIG. 6. This causes drill bit 7, guided by the inner wall of conical sleeve 15, to drill a hole 9 in bone 1 of which the hole's axis 21 will differ slightly from axis 20 of plate hole 3, as shown in FIG. 7. That is, axis 21 is angularly misaligned with axis 20. The difference between axis 21 of bone hole 9 (and thus the axis of locked screw 4 after insertion) and axis 20 of plate hole 3 is no more than, and preferably within, the tolerance angle, which assures satisfactory mechanical performance of the screw-plate coupling.

Locking screw 4 may then be imperfectly seated at a threaded hole 3 by placing shaft 6 in drilled hole 9. In other words, locking screw 4 is imperfectly seated at a threaded hole 3 when the thread on head 5 is offset from and only substantially parallel to the thread of threaded hole 3. Locking screw 4 may then be screwed into threaded hole 3 and drilled hole 9 by substantially engaging the thread on head 5 with the thread of threaded hole 3.

Therefore, positioning shaft 6 of bone screw 4 in drilled hole 9 ensures that the tolerance angle will not be exceeded when screwing screw 4 into a plate hole 3, thus avoiding the possibility of jamming the screw head into the plate and/or damaging the threads.

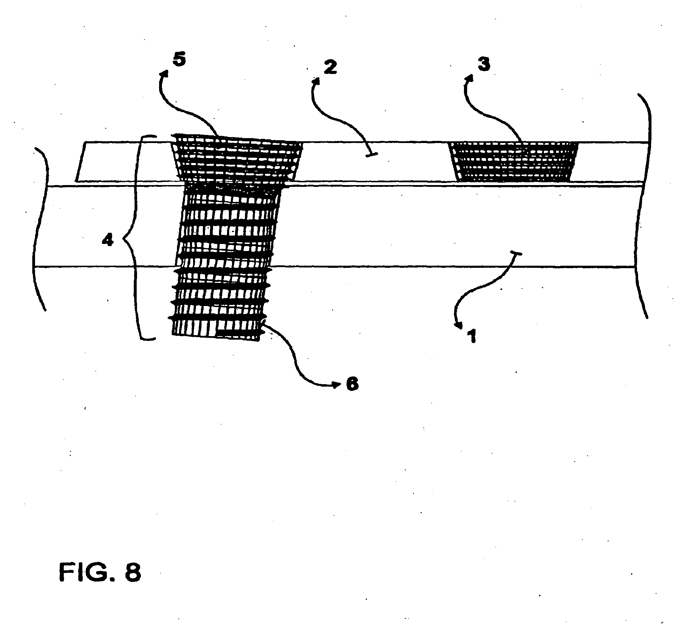

FIG. 8 shows locking screw 4 at its final position inside bone hole 9 with its axis slightly different (angularly misaligned) from the axis of plate hole 3.

The invention has been described in connection with the preferred embodiments. These embodiments, however, are merely for example and the invention is snot restricted to them. It will be understood by those skilled in the art that other variations and modifications can be easily made within the scope of the invention as defined by the appended claims. Therefore, the invention is only intended to be limited by the following claims.

## Claims

1. A bone plate system comprising:
A) a bone plate having an upper surface, a lower surface, and a hole extending through the upper and lower surfaces, the hole having a central axis; and
B) a drilling sleeve (15) comprising an elongated body having a conically-shaped bore (17) extending longitudinally there through and forming an inside wall in the body, the body having an end at the narrowest portion of the bore (17) configured to attach to the hole,
**characterized in that**
C) the hole of said plate is threaded;
D) the end of the body of the drilling sleeve (15) at the narrowest portion of the bore (17) has an external thread thereon that mates with the thread of the threaded hole of the bone plate; and
E) the threaded hole is conically shaped.

2. The system of claim 1 further comprising a bone plate screw having a head with an external thread thereon that mates with the thread of the threaded hole, the bone plate screw further comprising a threaded shank extending from the head.

3. The system of claim 2, wherein the bone plate screw has a central axis angularly misaligned with the central axis of the threaded hole when head of the bone plate screw is screwed into the threaded hole.

4. The system of claim 2, wherein the head of the bone plate screw is conically shaped.

5. The system of claim 1 wherein the central axis of the threaded hole is orthogonal to the upper surface.

6. The system of claim 1 further comprising a plurality of threaded holes extending through the upper and lower surfaces.

7. The system of claim 2, wherein
- the thread of the threaded hole has a tolerance angle at or within which said bone plate screw having a head with a corresponding external thread thereon can be imperfectly seated at the threaded hole and screwed into and out of the threaded hole, the bone plate screw having a central axis that coincides with the central axis of the threaded hole when perfectly seated and screwed into the threaded hole; and wherein:
- with the sleeve screwed into the threaded hole, the inner wall forms an angle with the central axis of the threaded hole that is equal to or less than the tolerance angle.

8. The system of claim 7, wherein the bone plate screw has a central axis angularly misaligned with the central axis of the threaded hole when head of the bone plate screw is screwed into the threaded hole.

9. The system of claim 7, wherein the bone plate screw is perfectly seated at the threaded hole when the thread of the bone plate screw is parallel to and offset from the thread of the threaded hole.

10. The system of claim 7, wherein the bone plate screw is imperfectly seated at the threaded hole when the thread of the bone plate screw is offset from and only substantially parallel to the thread of the threaded hole.

## Patentansprüche

1. Knochenplatten-System umfassend:
A) eine Knochenplatte mit einer oberen Fläche, einer unteren Fläche und einem sich durch die obere und untere Fläche erstreckenden Loch, wobei das Loch eine Zentralachse hat; und
B) eine Bohrhülse (15), welche einen longitudinalen Körper mit einer sich longitudinal durch diesen erstreckenden und eine Innenwand im Körper bildenden konisch ausgebildeten Bohrung (17) umfasst, wobei der Körper ein Ende am engsten Abschnitt der Bohrung (17) aufweist, welches zur Befestigung im Loch ausgebildet ist,
**dadurch gekennzeichnet, dass**
C) das Loch der Platte ein Gewinde aufweist;
D) das Ende des Körpers der Bohrhülse (15) am engsten Abschnitt der Bohrung (17) ein Aussengewinde aufweist, welches mit dem Gewinde des ein Gewinde aufweisenden Lochs der Knochenplatte zusammenpasst; und
E) das ein Gewinde aufweisende Loch konisch ausgebildet ist.

2. System nach Anspruch 1, **dadurch gekennzeichnet, dass** es zusätzlich eine Knochenplattenschraube umfasst, welche einen Kopf mit einem zu dem Gewinde des mit einem Gewinde versehenen Lochs passenden Aussengewinde hat, wobei die Knochenplattenschraube zusätzlich einen mit einem Gewinde versehenen Schaft umfasst, welcher sich vom Kopf erstreckt.

3. System nach Anspruch 2, **dadurch gekennzeichnet, dass** die Knochenplattenschraube eine Zentralachse aufweist, welche gegenüber der Zentralachse des mit einem Gewinde versehenen Lochs abgewinkelt ist, wenn der Kopf der Knochenplattenschraube in das mit einem Gewinde versehene Loch eingeschraubt ist.

4. System nach Anspruch 2, **dadurch gekennzeichnet, dass** der Kopf der Knochenplattenschraube konisch ausgebildet ist.

5. System nach Anspruch 1, **dadurch gekennzeichnet, dass** die Zentralachse des mit einem Gewinde versehenen Lochs orthogonal zur oberen Fläche ist.

6. System nach Anspruch 1, **dadurch gekennzeichnet, dass** es zusätzlich mehrere mit einem Gewinde versehene Löcher aufweist, welche sich durch die obere und untere Fläche erstrecken.

7. System nach Anspruch 2, **dadurch gekennzeichnet, dass**
- das Gewinde des ein Gewinde aufweisenden Lochs einen Toleranzwinkel hat, bei welchem oder innerhalb welchem die Knochenplattenschraube, welche einen Kopf mit einem korrespondierenden Aussengewinde aufweist, fehlerhaft in das eine Gewinde aufweisende Loch eingefügt werden kann und in das oder aus dem mit einem Gewinde versehenen Loch geschraubt werden kann, wobei die Knochenplattenschraube eine Zentralachse aufweist, welche mit der Zentralachse des mit einem Gewinde versehenen Lochs zusammenfällt, wenn diese perfekt in das mit einem Gewinde versehene Loch eingefügt und eingeschraubt wird; und
- bei in das mit einem Gewinde versehene Loch eingeschraubter Hülse, die Innenwand mit der Zentralachse des mit einem Gewinde versehenen Lochs einen Winkel einschliesst, welcher gleich oder kleiner als der Toleranzwinkel ist.

8. System nach Anspruch 7, **dadurch gekennzeichnet, dass** die Knochenplattenschraube eine Zentralachse aufweist, welche gegenüber der Zentralachse des mit einem Gewinde versehenen Lochs abgewinkelt ist, wenn der Kopf der Knochenplattenschraube in das mit einem Gewinde versehene Loch eingeschraubt ist.

9. System nach Anspruch 7, **dadurch gekennzeichnet, dass** die Knochenplattenschraube perfekt in das mit einem Gewinde versehene Loch eingefügt ist, wenn das Gewinde der Knochenplattenschraube parallel und versetzt zu dem Gewinde des ein Gewinde aufweisenden Lochs ist.

10. System nach Anspruch 7, **dadurch gekennzeichnet, dass** die Knochenplattenschraube fehlerhaft in das eine Gewinde aufweisende Loch eingefügt ist, wenn das Gewinde der Knochenplattenschraube versetzt und nur im wesentlichen parallel zu dem Gewinde des mit einem Gewinde versehenen Lochs ist.

## Revendications

1. Système de plaque d'ostéosynthèse comprenant :
A) une plaque d'ostéosynthèse présentant une surface supérieure, une surface inférieure et un trou s'étendant à travers les surfaces supérieure et inférieure, le trou présentant un axe central ; et
B) une douille de perçage (15) comprenant un corps allongé présentant un alésage de forme conique (17) s'étendant longitudinalement à travers celui-ci et formant une paroi interne à l'intérieur du corps, le corps présentant une extrémité, au niveau de la partie la plus étroite de l'alésage (17), conçue pour se fixer au trou,
**caractérisé en ce que**
C) le trou de ladite plaque est taraudé ;
D) l'extrémité du corps de la douille de perçage (15), au niveau de la partie la plus étroite de l'alésage (17), présente un filetage extérieur qui coopère avec le filetage du trou taraudé de la plaque d'ostéosynthèse ; et
E) le trou taraudé est de forme conique.

2. Système selon la revendication 1, comprenant en outre une vis de plaque d'ostéosynthèse comprenant une tête présentant un filetage extérieur qui coopère avec le filetage du trou taraudé, la vis de plaque d'ostéosynthèse comprenant en outre une tige filetée s'étendant depuis la tête.

3. Système selon la revendication 2, dans lequel la vis de plaque d'ostéosynthèse présente un axe central désaligné angulairement par rapport à l'axe central du trou taraudé lorsque la tête de la vis de plaque d'ostéosynthèse est vissée dans le trou taraudé.

4. Système selon la revendication 2, dans lequel la tête de la vis de plaque d'ostéosynthèse est de forme conique.

5. Système selon la revendication 1, dans lequel l'axe central du trou taraudé est orthogonal à la surface supérieure.

6. Système selon la revendication 1, comprenant en outre une pluralité de trous taraudés s'étendant à travers les surfaces supérieure et inférieure.

7. Système selon la revendication 2, dans lequel :
- le filetage du trou taraudé présente un angle de tolérance au niveau duquel ou à l'intérieur duquel ladite vis de plaque d'ostéosynthèse, qui présente une tête comprenant un filetage extérieur correspondant, peut être imparfaitement logée au niveau du trou taraudé et vissée dans le trou taraudé et dévissée de celui-ci, la vis de plaque d'ostéosynthèse présentant un axe central qui coïncide avec l'axe central du trou taraudé lorsque celle-ci est parfaitement logée et vissée dans le trou taraudé ; et
**dans lequel :**
- lorsque la douille est vissée dans le trou taraudé, la paroi interne forme un angle avec l'axe central du trou taraudé qui est inférieur ou égal à l'angle de tolérance.

8. Système selon la revendication 7, dans lequel la vis de plaque d'ostéosynthèse présente un axe central désaligné angulairement par rapport à l'axe central du trou taraudé lorsque la tête de la vis de plaque d'ostéosynthèse est vissée dans le trou taraudé.

9. Système selon la revendication 7, dans lequel la vis de plaque d'ostéosynthèse est parfaitement logée au niveau du trou taraudé lorsque la tête de la vis de plaque d'ostéosynthèse est parallèle à et désaxée par rapport au filetage du trou taraudé.

10. Système selon la revendication 7, dans lequel la vis de plaque d'ostéosynthèse est imparfaitement logée au niveau du trou taraudé lorsque la tête de la vis de plaque d'ostéosynthèse est désaxée par rapport et seulement essentiellement parallèle au filetage du trou taraudé.
